# EUROPEAN PATENT APPLICATION

(11) **EP 4 692 787 A1**
(43) Date of publication of application: **11.02.2026**
(21) Application number: 24780816.5
(22) Date of filing: 29.03.2024
(51) Int. Cl.: G01N 31/00, G01N 21/33, G01N 21/61, G01N 27/08, G01N 33/18

(54) **MEASUREMENT METHOD AND MEASUREMENT DEVICE FOR NITROGEN/CARBON-CONTAINING COMPOUND**

(30) Priority: 31.03.2023 JP 2023058354
(71) Applicant: National Institute of Advanced Industrial Science and Technology, Chiyoda-ku Tokyo 100-8921 (JP)
(72) Inventor: NAKAZATO Tetsuya, Tsukuba-shi, Ibaraki 305-8560 (JP)
(74) Representative: V.O.
(86) International application number: PCT/JP2024/013060
(87) International publication number: WO 2024/204721

(57) **Abstract**

The present invention provides a measurement method that enables general-purpose quantitative analysis capable of quantifying all nitrogen/carbon-containing compounds contained in water with a calibration curve based on a single compound. In addition, the invention provides a direct, inexpensive, rapid, and simple method for measuring cyanide in water by spectrophotometry or electrical conductivity measurement. The measurement method for nitrogen/carbon-containing compounds is a method for measuring a compound containing at least one element selected from the group consisting of nitrogen and carbon. The method comprises: a photoreaction step of irradiating an aqueous solution containing the nitrogen/carbon-containing compound with light having a wavelength of 190 nm or less to generate analyte ions; and a measurement step of measuring the analyte ions. The analyte ions are at least one ion selected from the group consisting of nitrate ions, nitrite ions, and carbonate ions.

## Description

### TECHNICAL FIELD

The present invention relates to a measurement method and a measurement device for a nitrogen/carbon-containing compound.

This application claims priority based on Japanese Patent Application No. 2023-058354, filed in Japan on March 31, 2023, the content of which is incorporated herein by reference.

### BACKGROUND ART

Conventionally, for the detection and quantitative analysis of nitrogen/carbon-containing compounds, methods such as absorbance photometry or fluorescence photometry are feasible when the compounds possess specific and selectively detectable physical properties, such as spectroscopic characteristics. However, for compounds lacking such selectively detectable properties, particularly cyanide, conventional methods involve using reagents to induce color development, followed by measurement with absorbance photometry or fluorescence photometry. However, there is no simple measurement method capable of measuring all types of nitrogen/carbon-containing compounds.

In absorbance photometry, cyanide ions are converted to cyanogen chloride using a chloramine T solution, then developed with a mixed solution of 4-pyridinecarboxylic acid and pyrazolone, and measured at 638 nm (Non-Patent Document 1). Other colorimetric reagents include a mixed solution of pyridine and pyrazolone or a mixed solution of pyridine and barbituric acid. In fluorescence photometry, a method using o-phthalaldehyde solution as a colorimetric reagent is also available (Non-Patent Document 2). All these methods require expensive, hazardous, or unstable reagents. Additionally, there are methods that combine absorbance photometry or fluorescence photometry with ion chromatography to detect cyanide by separating coexisting substances.

In addition, electrochemical detection methods exist, including ion electrode methods using silver iodide or the like as the sensing membrane (Non-Patent Document 3), polarography (Non-Patent Document 4), and a combination of ion chromatography with constant-potential coulometry (Non-Patent Document 5). While electrochemical detection methods do not require colorimetric reagents, they use expensive noble metals such as silver or toxic mercury in the electrodes. Furthermore, there is a method combining ion chromatography with conductivity detection. Since hydrogen cyanide is a weak acid and hardly dissociates into cyanide ions in water, conductivity detection has low sensitivity. Therefore, methods such as converting cyanide to thiocyanate ions using polysulfides for detection (Non-Patent Document 6), or converting it to cyanate ions using hypochlorite or chloramine T for detection (Non-Patent Documents 7 and 8) are used. However, these methods require expensive, unstable, or hazardous oxidizing reagents.

There are also methods to convert cyanide into a measurable chemical form without using oxidizing reagents. For example, a method involves converting cyanide to cyanate ions using a batch-type ultraviolet irradiator with a high-pressure mercury lamp, followed by collection and detection by ion chromatography conductivity detection (Non-Patent Document 9). Although this method does not use colorimetric reagents, the batch-type ultraviolet irradiation process is complex and requires lengthy operations, approximately one hour. Moreover, the conversion rate varies depending on the type of cyanide, and the detection method is limited to conductivity detection.

Furthermore, there are methods that combine ion chromatography with amperometry for detection (Non-Patent Documents 10 and 11). These methods use a flow-type ultraviolet irradiator, in which a Pyrex (registered trademark) glass tube or PTFE tube is wound around a high-pressure mercury lamp, to decompose cyanide into cyanide ions, which are then detected to measure cyanide. Although these methods do not use slump colorimetric reagents, the flow-type ultraviolet irradiation process is limited to conversion to cyanide ions and requires electrochemical detection methods using expensive electrodes.

Additionally, there is a method using a flow-type ultraviolet irradiator wound with a PTFE tube to decompose cyanide into cyanide ions, which are then vaporized as hydrogen cyanide using an acid reagent and detected by photometry using cellulose tape impregnated with a mixed solution of N,N-dimethyl-p-phenylenediamine (DPD) sulfate and copper(II) acetate (Non-Patent Document 12). This flow-type ultraviolet irradiation process is limited to conversion to cyanide ions and requires expensive reagents for detection.

Other methods include gas chromatography (Non-Patent Documents 13, 14, and 15). However, cyanide ions cannot be directly measured. Therefore, cyanide ions are converted to hydrogen cyanide, halogenated cyanide, or volatile organic derivatives using acids, halogenating reagents, or derivatizing organic reagents, and detected by flame ionization detection (FID), electron capture detection (ECD), or mass spectrometry (MS). These methods all require expensive or unstable reagents or costly analytical instruments.

For other nitrogen/carbon-containing compounds lacking selectively detectable properties, similar to cyanide-containing compounds, there are analytical methods using colorimetric reagents specific to individual compounds (Non-Patent Document 16). However, colorimetric reagents are expensive, and operations such as color development reactions are complex. Moreover, quantitative analysis of nitrogen/carbon-containing compounds requires standard samples for calibration curves specific to each compound. All these conventional methods require expensive reagents and are difficult to apply to the detection and quantification of all carbon and nitrogen-containing compounds.

Additionally, there are methods for separately quantifying nitrogen and carbon components in compounds. One method involves oxidizing nitrogen/carbon-containing compounds in sample water at high temperatures using a combustion furnace filled with a noble metal catalyst to produce nitrogen oxides and carbon dioxide, which are detected by ozone chemiluminescence and infrared absorbance photometry (Non-Patent Document 3). However, this method requires expensive catalysts, and the catalyst and combustion furnace suffer from long-term instability due to degradation from high-temperature use. For the analysis of individual carbon-containing organic compounds in water, a method involves separation by size-exclusion chromatography, followed by oxidative decomposition under ultraviolet irradiation in the presence of oxidizing reagents or catalysts such as titanium, converting them to carbon dioxide for detection and quantification by infrared absorbance photometry (Non-Patent Documents 17 and 18). However, this method requires expensive reagents or catalysts. Moreover, this method cannot be applied to the detection and quantification of nitrogen-containing compounds, including cyanide, or the simultaneous detection and quantification of nitrogen and carbon components in nitrogen/carbon-containing compounds.

There is also a method for the simultaneous detection and quantification of nitrogen and carbon components in nitrogen/carbon-containing compounds (Non-Patent Document 19). This method involves separating compounds with single bonds between carbon and nitrogen, such as urea or amino acids in water, using size-exclusion chromatography, splitting the eluate into two streams. One stream is introduced into a spiral quartz tube placed outside a low-pressure mercury lamp, and the other is introduced into a thin-layer quartz container placed outside the same lamp. Vacuum ultraviolet light is irradiated to decompose the single-bonded nitrogen and carbon compounds into nitrate ions and carbon dioxide, which are detected by ultraviolet absorbance photometry and infrared absorbance photometry, respectively. However, the spiral tube photoreactor for nitrogen measurement has the reaction tube placed outside the lamp, resulting in light irradiation only from one side of the tube, and the light intensity is attenuated by the surrounding air before reaching the tube, leading to low irradiation efficiency and decomposition efficiency. Consequently, a long reaction tube of 4 meters is used, requiring a reaction time of approximately 30 minutes to convert to nitrate ions. This method is applicable only to the decomposition of easily degradable single-bonded carbon and nitrogen compounds and is not suitable for converting refractory nitrogen and carbon compounds with multiple bonds, such as cyanide, into nitrogen oxoanions as final products, particularly for short-time applications essential for online measurement. Additionally, it requires complex device configurations and operations. For example, the system requires two large photoreactors using 1-meter low-pressure mercury lamps, and the thin-layer photoreactor for carbon measurement has a complex multi-tube structure. It also necessitates nitrogen gas to suppress light attenuation from the light source to the thin-layer photoreactor and for cooling the light source, as well as a stirrer to promote gas-phase separation of generated carbon dioxide.

### Citation List

### Non-Patent Documents

[Non-Patent Document 1] Keiichiro Ishii, Takeharu Iwamoto, Kazuhiko Yamanishi: Japan Analyst, 1973, vol. 22, pp. 448-450
[Non-Patent Document 2] A. Sano, M. Takezawa and S. Takitani: Analytical Sciences, October 1986, vol. 2, pp. 491-492
[Non-Patent Document 3] Factory Wastewater Testing Method JIS K 0102:2019 (2019)
[Non-Patent Document 4] J. H. Karchmer and M. T. Walker: Analytical Chemistry, January 18, 1955, vol. 27, no. 1, pp. 37-41
[Non-Patent Document 5] J. E. Girard: Analytical Chemistry, June 1979, vol. 51, no. 7, pp. 836-839
[Non-Patent Document 6] Hiroshi Satake, Hiroaki Sorakawa, Sanae Ikeda: The Chemical Society of Japan, 1988, no. 9, pp. 1587-1590
[Non-Patent Document 7] M. Nonomura: Analytical Chemistry, 1987, vol. 59, no. 17, pp. 2073-2076
[Non-Patent Document 8] M. Nonomura, T. Hobo: Journal of Chromatography, 1989, vol. 465, pp. 395-401
[Non-Patent Document 9] Makoto Nonomura, Masanori Takakamo: Journal of Industrial Water, October 1997, no. 469, pp. 16-21
[Non-Patent Document 10] Y. Liu, R. D. Rocklin, R. J. Joyce and M. J. Doyle: Analytical Chemistry, 1990, vol. 62, pp. 766-770
[Non-Patent Document 11] L. Solujic, E. B. Milosavljevic and M. R. Straka: Analyst, 1999, vol. 124, pp. 1255-1260
[Non-Patent Document 12] Takakazu Hanzawa, Chino Nagashima, Yoshinori Kamatani, Manabu Ishiguro, Nobuo Nakano: Analytical Chemistry, 2006, vol. 55, no. 10, pp. 773-779
[Non-Patent Document 13] C. R. Schneider and H. Freund: Analytical Chemistry, January 1962, vol. 34, no. 1, pp. 69-74
[Non-Patent Document 14] G. Nota, R. Palombari and C. Improta: Journal of Chromatography, 1976, vol. 123, pp. 411-413
[Non-Patent Document 15] Y. Noda: Bulletin of the Society of Sea Water Science, Japan, 2013, vol. 67, pp. 224-228
[Non-Patent Document 16] R. L. Cunico and T. Schlabach: Journal of Chromatography, 1983, vol. 266, pp. 461-470
[Non-Patent Document 17] Nobuyuki Kawasaki, Kazuo Matsushige, Akio Imai, Kazuhiro Komatsu, Fumikazu Ogishi, Masato Yahata, Hirohisa Mikami, Takeshi Goto: Abstracts of The Japanese Society of Limnology, 2007, vol. 72, p. 80
[Non-Patent Document 18] N. Her, G. Amy, D. Foss, J. Cho, Y. Yoon, P. Kosenka: Environmental Science & Technology, 2002, vol. 36, pp. 1069-1076
[Non-Patent Document 19] S. A. Huber, A. Balz, M. Abert, W. Pronk: Water Research, 2011, vol. 45, pp. 879-885

### SUMMARY OF INVENTION

### Technical Problem

There is a need for a method to measure the nitrogen and carbon components contained in all types of nitrogen/carbon-containing compounds. In particular, cyanide, which is highly toxic, is one of the critical substances to be monitored for the safety management of environmental water, tap water, industrial water, wastewater, and soil. Thus, there is a demand for an inexpensive, rapid, and simple measurement method for nitrogen/carbon-containing compounds such as cyanide. Since cyanide commonly contains cyano, selective detection of cyano would be desirable. However, cyano lacks properties that allow selective and highly sensitive detection. Consequently, measurements are conducted using colorimetric reagents for absorbance or fluorescence photometry, or electrochemical detection methods using noble metal electrodes. However, these methods require expensive, unstable, or hazardous reagents or materials and involve complex operations. Additionally, coexisting substances in the water sample may adversely affect color development or electrode reactions, limiting the applicability to actual water samples. As a measurement method that does not use colorimetric reagents or electrodes, there is a method of converting cyanide ions into cyanate ions by ultraviolet irradiation and detecting them by ion chromatography conductivity detection. However, this method cannot achieve inexpensive, rapid, and simple measurement of cyano in water. This is because the ultraviolet irradiation process is batch-type, requiring complex and time-consuming operations. Furthermore, the conversion rate varies depending on the type of cyanide, and the detection method is limited to conductivity detection, which lacks selectivity.

The inventors of the present invention have succeeded in measuring nitrogen/carbon-containing compounds such as cyanide by irradiating an aqueous solution of nitrogen/carbon-containing compounds with light having a wavelength of 190 nm or less to generate nitrate ions, nitrite ions, and carbonate ions, and measuring these ions.

The present invention provides a measurement method and device enabling general-purpose quantitative analysis of all nitrogen/carbon-containing compounds in water using a calibration curve of a single compound.

Additionally, it provides a method and device for directly, inexpensively, rapidly, and simply measuring cyanide in water using absorbance photometry or conductivity detection.

### Solution to Problem

The present invention has been completed based on these findings and includes the following aspects:
[1] A method for measuring a nitrogen/carbon-containing compound comprising at least one element selected from the group consisting of nitrogen and carbon, the method comprising:
   a photoreaction step of irradiating an aqueous solution containing the nitrogen/carbon-containing compound with light having a wavelength of 190 nm or less to generate analyte ions; and
   a measurement step of measuring the analyte ions,
   wherein the analyte ions are at least one ion selected from the group consisting of nitrate ions, nitrite ions, and carbonate ions.
[2] The method for measuring a nitrogen/carbon-containing compound according to [1], wherein the nitrogen/carbon-containing compound is cyanide.
[3] The method for measuring a nitrogen/carbon-containing compound according to [1] or [2], wherein the measurement step includes a nitrogen component measurement step of measuring the nitrate ions and the nitrite ions by ultraviolet absorbance photometry.
[4] The method for measuring a nitrogen/carbon-containing compound according to any one of [1] to [3], wherein the measurement step includes a nitrogen component measurement step of measuring the nitrate ions and the nitrite ions by conductivity detection.
[5] The method for measuring a nitrogen/carbon-containing compound according to any one of [1] to [4], wherein the measurement step includes a carbon component measurement step of converting the carbonate ions into carbon dioxide and measuring the carbon dioxide by infrared absorbance photometry.
[6] A measurement device for measuring a nitrogen/carbon-containing compound comprising at least one element selected from the group consisting of nitrogen and carbon, the device comprising:
   a photoreaction unit and a measurement unit,
   wherein the photoreaction unit comprises a reaction vessel and a light source, the light source irradiating light having a wavelength of 190 nm or less,
   the photoreaction unit generates analyte ions from an aqueous solution containing the nitrogen/carbon-containing compound,
   the measurement unit measures the analyte ions, and
   the analyte ions are at least one ion selected from the group consisting of nitrate ions, nitrite ions, and carbonate ions.
[7] The measurement device for a nitrogen/carbon-containing compound according to [6], wherein the nitrogen/carbon-containing compound is cyanide.
[8] The measurement device for a nitrogen/carbon-containing compound according to [6] or [7], wherein the measurement unit comprises a nitrogen component measurement unit comprising an ultraviolet absorbance photometer.
[9] The measurement device for a nitrogen/carbon-containing compound according to any one of [6] to [8], wherein the measurement unit comprises a nitrogen component measurement unit comprising a conductivity detector.
[10] The measurement device for a nitrogen/carbon-containing compound according to any one of [6] to [9], wherein the measurement unit comprises a carbon component measurement unit comprising a conversion unit that converts the carbonate ions into carbon dioxide and an infrared absorbance photometer.
[11] The measurement device for a nitrogen/carbon-containing compound according to any one of [6] to [10], wherein the measurement device is a flow-type measurement device, further comprising a liquid delivery unit and a sample introduction unit, and the liquid delivery unit, the sample introduction unit, the photoreaction unit, and the measurement unit are arranged in this order.
[12] The measurement device for a nitrogen/carbon-containing compound according to any one of [6] to [10], wherein the measurement device is a flow-type measurement device, further comprising a liquid delivery unit, a sample introduction unit, and a separation column, and the liquid delivery unit, the sample introduction unit, the separation column, the photoreaction unit, and the measurement unit are arranged in this order.
[13] The measurement device for a nitrogen/carbon-containing compound according to any one of [6] to [10], wherein the measurement device is a flow-type measurement device, further comprising a liquid delivery unit, a sample introduction unit, and a suppressor, and the liquid delivery unit, the sample introduction unit, the suppressor, the photoreaction unit, and the measurement unit are arranged in this order.
[14] The measurement device for a nitrogen/carbon-containing compound according to any one of [6] to [13], wherein the photoreaction unit further includes a temperature control unit.
[15] The measurement device for a nitrogen/carbon-containing compound according to [14], wherein the temperature control unit includes a temperature measurement device for measuring the temperature of the photoreaction unit and a cooling device for cooling the photoreaction unit, and wherein the temperature of the photoreaction unit is at least one temperature selected from the group consisting of the temperature of the reaction vessel, the temperature of the light source, and the temperature of the aqueous solution.

### Advantageous Effects of Invention

The present invention provides a measurement method and device enabling general-purpose quantitative analysis of all nitrogen/carbon-containing compounds in water using a calibration curve of a single compound.

Additionally, it provides a method and device for directly, inexpensively, rapidly, and simply measuring cyanide in water using absorbance photometry or conductivity detection.

### BRIEF DESCRIPTION OF DRAWINGS

[Figure 1] A schematic diagram showing the measurement device of the first embodiment.
[Figure 2] A schematic diagram showing the measurement device of the second embodiment using an ultraviolet absorbance photometer.
[Figure 3] A schematic diagram showing the measurement device of the third embodiment using ultraviolet and infrared absorbance photometers.
[Figure 4] A schematic diagram showing the measurement device of the third embodiment using a conductivity detector and an infrared absorbance photometer.
[Figure 5] A schematic diagram showing the measurement device of the fourth embodiment using an ion chromatograph.
[Figure 6] A schematic diagram showing the measurement device of the fifth embodiment using a suppressor.
[Figure 7] A diagram showing the measurement results of Example 1 and Comparative Example 1.
[Figure 8] A diagram showing the measurement results of Example 1.
[Figure 9] A diagram showing the measurement results of Example 2 and Comparative Example 2.
[Figure 10] A diagram showing the measurement results of Example 3 and Comparative Example 3.
[Figure 11] A diagram showing the measurement results of Example 4.
[Figure 12] A diagram showing the measurement results of Example 5 (with irradiation).
[Figure 13] A diagram showing the measurement results of Comparative Example 4 (without irradiation).
[Figure 14] A diagram showing the measurement results of Example 6 (with irradiation).
[Figure 15] A diagram showing the measurement results of Comparative Example 5 (without irradiation).

### DESCRIPTION OF EMBODIMENTS

Hereinafter, the measurement method and measurement device for a nitrogen/carbon-containing compound according to embodiments of the present invention will be described in detail. However, the present invention is not limited to the following embodiments.

### (Explanation of Terms)

The term "nitrogen/carbon-containing compound" in the present invention encompasses, for example, cyanides such as heavy metal cyano complex compounds, "nitrogen/carbon-containing ions" such as cyanide ions, and "nitrogen/carbon-containing materials."

### (Measurement Method for Nitrogen/Carbon-Containing Compound)

The measurement method for a nitrogen/carbon-containing compound according to one embodiment of the present invention (hereinafter sometimes referred to as the measurement method of the present embodiment) is a method for measuring a nitrogen/carbon-containing compound containing at least one element selected from the group consisting of nitrogen and carbon. The method comprises: a photoreaction step of irradiating an aqueous solution containing the nitrogen/carbon-containing compound with light having a wavelength of 190 nm or less to generate analyte ions; and a measurement step of measuring the analyte ions, wherein the analyte ions include at least one ion selected from the group consisting of nitrate ions, nitrite ions, and carbonate ions.

The measurement step may include a nitrogen component measurement step of measuring the nitrate ions and the nitrite ions using ultraviolet absorbance photometry, and may include a nitrogen component measurement step of measuring the nitrate ions and the nitrite ions using conductivity detection. Additionally, the measurement step may include, after the nitrogen component measurement step, a carbon component measurement step of converting the carbonate ions into carbon dioxide and measuring the carbon dioxide using infrared absorbance photometry. Preferably, the measurement step includes both the nitrogen component measurement step and the carbon component measurement step.

The light having a wavelength of 190 nm or less may be vacuum ultraviolet light (Vacuum UV, VUV) in the wavelength range of 100 nm to 190 nm. For example, when a low-pressure mercury lamp is used as the light source, light around 185 nm may be used, and when a Xe excimer lamp is used, light around 172 nm may be used. In addition to the light having a wavelength of 190 nm or less, light having a wavelength exceeding 190 nm but not more than 300 nm, such as deep ultraviolet light (Deep-Violet DUV), may also be irradiated. For example, when a low-pressure mercury lamp is used as the light source, light around 254 nm may be used. When light exceeding 190 nm but not more than 300 nm is also irradiated, ozone is generated from dissolved oxygen in the aqueous sample, allowing ultraviolet decomposition to occur concurrently. This enables the oxidative decomposition of readily degradable intermediate decomposition products obtained from the oxidative decomposition of refractory nitrogen/carbon-containing compounds by the photoreaction with light of 190 nm or less, thereby promoting conversion to the final products, namely nitrate ions, nitrite ions, and carbonate ions.

### [Nitrogen/carbon-containing Compound]

The nitrogen/carbon-containing compound to be measured by the measurement method of the present embodiment may be a compound that does not contain carbon or a compound that does not contain nitrogen. Preferably, it is a compound containing both nitrogen and carbon. Examples of nitrogen/carbon-containing compounds include cyanides (cyan compounds), cyanide ions, nitro compounds, nitrogen-containing organic chelate compounds, amino acids, proteins, ethylenediaminetetraacetic acid (EDTA), humic substances, pesticides, and purification agents. From the viewpoint of lacking selectively detectable physical properties, the effect of the measurement method of the present embodiment is particularly remarked when the nitrogen/carbon-containing compound is a cyanide (cyan compound), a cyanide ion, or a substance containing the same.

The measurement method of the present embodiment will be described below using the measurement method for cyanide as an example.

### [Measurement Method for Cyanide]

The measurement method for cyanide according to the present embodiment comprises: a photoreaction step of irradiating an aqueous solution containing cyanide with light having a wavelength of 190 nm or less to generate analyte ions; and a measurement step of measuring the analyte ions, wherein the analyte ions include at least one ion selected from the group consisting of nitrate ions, nitrite ions, and carbonate ions.

The measurement step may include a nitrogen component measurement step of measuring the nitrate ions and the nitrite ions using ultraviolet absorbance photometry, and may include a nitrogen component measurement step of measuring the nitrate ions and the nitrite ions using conductivity detection. Additionally, the measurement step may include, after the nitrogen component measurement step, a carbon component measurement step of converting the carbonate ions into carbon dioxide and measuring the carbon dioxide using infrared absorbance photometry. Preferably, the measurement step includes both the nitrogen component measurement step and the carbon component measurement step.

The measurement method for cyanide according to the present embodiment can be performed using the measurement device described below.

The measurement method for cyanide according to the present embodiment is a method capable of measuring highly toxic cyanide in water without using reagents. As described in the embodiments of the measurement device below, this method includes a step of efficiently irradiating the water sample with vacuum ultraviolet light using, for example, a high-efficiency vacuum ultraviolet photoreactor. This is considered to generate hydroxyl radicals (•OH) efficiently from water molecules in the sample, which are used as an oxidizing agent. Consequently, refractory cyanide is oxidatively decomposed into the final products, namely nitrate ions, nitrite ions, and carbonate ions, rapidly, for example, in a few minutes. This enables direct, inexpensive, rapid, and simple measurement using conventional absorbance photometry or conductivity detection without the use of colorimetric reagents or electrodes.

The measurement method for the analyte ions such as nitrate ions, nitrite ions, and carbonate ions is not particularly limited, and conventional measurement methods can be used. For example, conventional absorbance photometry or conductivity detection can be used.

The light having a wavelength of 190 nm or less has the same meaning as the light having a wavelength of 190 nm or less described in the section on the measurement method for nitrogen/carbon-containing compounds above. Similarly, in addition to the light having a wavelength of 190 nm or less, light having a wavelength exceeding 190 nm but not more than 300 nm, such as deep ultraviolet light (Deep-Violet DUV), may also be irradiated. When light exceeding 190 nm but not more than 300 nm is also irradiated, ozone is generated from dissolved oxygen in the aqueous sample, allowing ultraviolet decomposition to occur concurrently. This enables the oxidative decomposition of readily degradable intermediate decomposition products, such as low-molecular-weight organic acids like acetic acid or nitrite ions, obtained from the oxidative decomposition of refractory nitrogen/carbon-containing compounds, including cyanide, by the photoreaction with light of 190 nm or less, thereby promoting conversion to the final products, namely nitrate ions, nitrite ions, and carbonate ions.

### (Measurement Device for Nitrogen/carbon-containing Compound)

The measurement device for a nitrogen/carbon-containing compound according to one embodiment of the present invention (hereinafter sometimes referred to as the measurement device of the present embodiment) is a device for measuring a nitrogen/carbon-containing compound containing at least one element selected from the group consisting of nitrogen and carbon. The device comprises a photoreaction unit and a measurement unit. The photoreaction unit includes a reaction vessel and a light source, wherein the light source irradiates light having a wavelength of 190 nm or less. Examples of the reaction vessel include a reaction tube and a thin-layer reactor. The photoreaction unit generates analyte ions from an aqueous solution containing the nitrogen/carbon-containing compound, and the measurement unit measures the analyte ions. The analyte ions include at least one ion selected from the group consisting of nitrate ions, nitrite ions, and carbonate ions.

The measurement unit may include a nitrogen component measurement unit comprising an ultraviolet absorbance photometer, and may include a nitrogen component measurement unit comprising a conductivity detector. Additionally, the measurement unit may include a carbon component measurement unit comprising a conversion unit that converts the carbonate ions into carbon dioxide and an infrared absorbance photometer. Preferably, the measurement device for the nitrogen/carbon-containing compound is a flow-type measurement device.

### The wavelength range may be from 100 nm to 190 nm.

Examples of the light source include a mercury lamp and a Xe excimer lamp. Examples of the lamp include a low-pressure mercury lamp with a power of 6 to 110 watts, preferably 40 watts or more.

The light source may irradiate not only light in the wavelength range of 190 nm or less but also deep ultraviolet light exceeding 190 nm but not more than 300 nm. For example, when a low-pressure mercury lamp is used as the light source, in addition to vacuum ultraviolet light around 185 nm, deep ultraviolet light around 254 nm may be irradiated. In this case, ozone is generated from dissolved oxygen in the aqueous sample, allowing ultraviolet decomposition to occur concurrently. This enables the oxidative decomposition of readily degradable intermediate decomposition products obtained from the oxidative decomposition of refractory nitrogen/carbon-containing compounds by the photoreaction with light of 190 nm or less, thereby promoting conversion to the final products, namely nitrate ions, nitrite ions, and carbonate ions.

Examples of the reaction tube include a quartz reaction tube. Synthetic fused silica capable of transmitting light of 190 nm or less is preferable.
Size: Inner diameter: 1 to 6 mm, preferably 1 to 2 mm, more preferably 1 mm or less
Length: 100 to 300 mm, preferably 200 to 300 mm
Shape: Cylindrical

Examples of the photoreaction unit include a built-in reaction tube photoreactor integrated within the lamp. An example of such a built-in reaction tube photoreactor is the one disclosed in Patent Document A below. Alternatively, a thin-layer reactor capable of transmitting light of 190 nm or less may be used, such as a reactor comprising a single synthetic fused silica plate and a glass plate with a flow path for introducing the sample liquid. The glass plate may also be a quartz plate.
[Patent Document A] Japanese Patent No. 3268447

The photoreaction unit may further include a temperature control unit. Preferably, the temperature control unit includes a temperature measurement device for measuring the temperature of the photoreaction unit and a cooling device for cooling the photoreaction unit. The temperature of the photoreaction unit may be at least one temperature selected from the group consisting of the temperature of the reaction vessel, the temperature of the light source, and the temperature of the aqueous solution.

Examples of the nitrogen component measurement unit comprising the ultraviolet absorbance photometer include a flow-type ultraviolet absorbance photometer.

Examples of the conversion unit that converts the carbonate ions into carbon dioxide include a pH buffer solution introduction unit for introducing a pH buffer solution and a glass gas-liquid separator.

For the pH buffer solution, a buffer solution with a pH of less than 2 is preferable to adjust the pH to 2 or less for the purpose of separating carbonate ions and dissolved carbon dioxide in the sample water after the photoreaction step into the gas phase. An example of the buffer solution includes 0.1 mol/L phosphoric acid. The introduction amount depends on the initial pH, pH buffering capacity, and the flow rate of the sample water. For example, when the initial pH of the sample water is neutral, with no pH buffering capacity and a flow rate of 1.0 mL/min, the introduction amount is at least 1/10 of the sample water volume, and the introduction rate is at least 0.1 mL/min.

Examples of the flow-type measurement device include a configuration in which the components are connected using Teflon (registered trademark) tubes.

The inner diameter of the Teflon tube is 0.1 mm to 1 mm, preferably 0.1 to 0.5 mm.

The flow rate depends on the performance of the photoreactor but, for the built-in reaction tube photoreactor, is, for example, 0.1 to 3 mL/min, preferably 0.1 to 1 mL/min.

The measurement device for the nitrogen/carbon-containing compound may be a flow-type measurement device, further comprising a liquid delivery unit and a sample introduction unit, with the liquid delivery unit, the sample introduction unit, the photoreaction unit, the nitrogen component measurement unit, and the carbon component measurement unit arranged in this order.

The measurement device for the nitrogen/carbon-containing compound may further comprise a liquid delivery unit, a sample introduction unit, and a separation column, with the liquid delivery unit, the sample introduction unit, the separation column, the photoreaction unit, and the measurement unit arranged in this order.

The measurement device for the nitrogen/carbon-containing compound may further comprise a liquid delivery unit, a sample introduction unit, and a suppressor, with the liquid delivery unit, the sample introduction unit, the suppressor, the photoreaction unit, and the measurement unit arranged in this order.

Examples of the liquid delivery unit include pumps such as a peristaltic tube pump, a plunger pump, or a syringe pump.

Examples of the separation column include an ion chromatograph. An example of the column used is the AS15 column manufactured by Thermo Scientific. An example of the mobile phase includes an aqueous potassium hydroxide solution.

### [Nitrogen/carbon-containing Compound]

The nitrogen/carbon-containing compound to be measured by the measurement device of the present embodiment has the same meaning as the nitrogen/carbon-containing compound to be measured by the measurement method of the present embodiment described above.

The measurement device of the present embodiment will be described below using the measurement device for cyanide as an example.

### [Measurement Device for Cyanide]

The measurement device for cyanide according to the present embodiment comprises a photoreaction unit and a measurement unit. The photoreaction unit includes a reaction vessel and a light source, wherein the light source irradiates light having a wavelength of 190 nm or less. Examples of the reaction vessel include a reaction tube and a thin-layer reactor. The photoreaction unit generates analyte ions from an aqueous solution containing the nitrogen/carbon-containing compound, and the measurement unit measures the analyte ions. The analyte ions include at least one ion selected from the group consisting of nitrate ions, nitrite ions, and carbonate ions.

The measurement device for cyanide according to the present embodiment has the same configuration as the measurement device for the nitrogen/carbon-containing compound.

The light source may irradiate not only light in the wavelength range of 190 nm or less but also deep ultraviolet light exceeding 190 nm but not more than 300 nm. For example, when a low-pressure mercury lamp is used as the light source, in addition to vacuum ultraviolet light around 185 nm, deep ultraviolet light around 254 nm may be irradiated. In this case, ozone is generated from dissolved oxygen in the aqueous sample, allowing ultraviolet decomposition to occur concurrently. This enables the oxidative decomposition of readily degradable intermediate decomposition products, such as low-molecular-weight organic acids like acetic acid or nitrite ions, obtained from the oxidative decomposition of refractory nitrogen/carbon-containing compounds, including cyanide, by the photoreaction with light of 190 nm or less, thereby promoting conversion to the final products, namely nitrate ions, nitrite ions, and carbonate ions.

Hereinafter, the first to fifth embodiments of the measurement method and measurement device for a nitrogen/carbon-containing compound will be described in detail with cyanide as an example of the measurement target. Additionally, the sixth embodiment of the measurement method and measurement device for a nitrogen/carbon-containing compound will be described in detail with p-nitrophenol as another example of the measurement target. In the sixth embodiment, the quantitative measurement method will also be described.

### [First Embodiment]

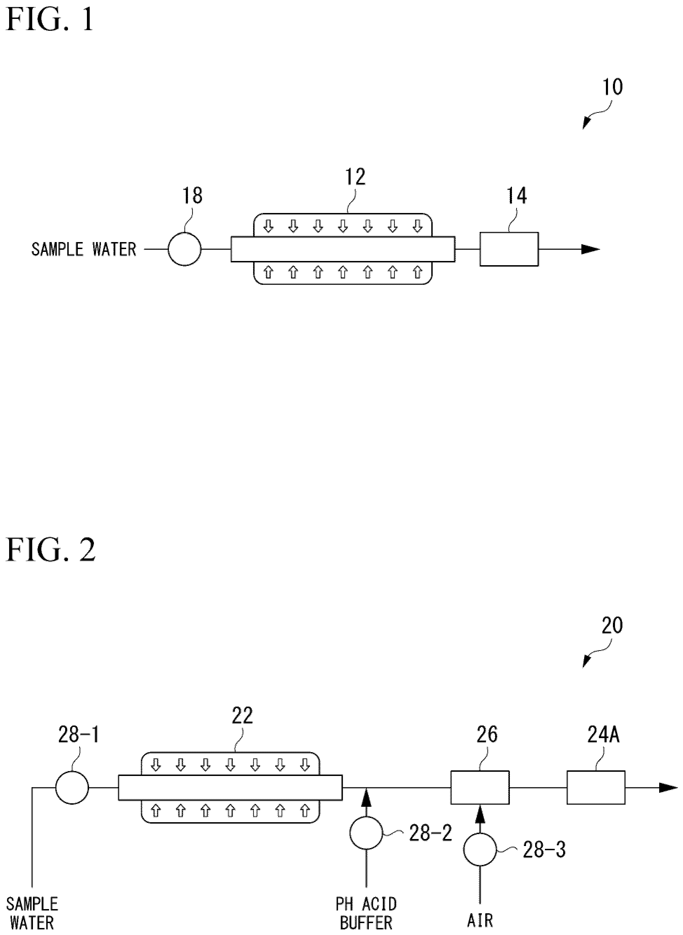

Figure 1 is a schematic diagram showing the measurement device of the first embodiment (cyanide measurement device 10).

The device 10 comprises a pump 18, a photoreaction unit (photoreactor 12), and a measurement unit (detector 14). The pump 18 may be a peristaltic tube pump. The photoreaction unit (photoreactor 12) may use a built-in reaction tube photoreactor that incorporates a quartz reaction tube within a 40-watt low-pressure mercury lamp as the light source. The measurement unit (detector 14) may use a flow-type ultraviolet absorbance photometer. These components are connected with Teflon tubes to form a flow-type measurement device.

The specific measurement method using the measurement device of Figure 1 will be described in Example 1 below.

### [Second Embodiment]

Figure 2 is a schematic diagram showing the measurement device of the second embodiment (cyanide measurement device 20).

The device 20, similar to Figure 1, comprises a pump 28, a photoreaction unit (photoreactor 22), and a measurement unit. However, unlike the flow-type ultraviolet absorbance photometer used in the first embodiment, the measurement unit uses a conversion unit that converts carbonate ions into carbon dioxide and an infrared absorbance photometer. The conversion unit that converts carbonate ions into carbon dioxide includes a pH buffer solution introduction unit (pump 28-2) and a gas-liquid separation unit (air introduction pump 28-3 and gas-liquid separator 26). Specifically, the device 20 is a flow-type measurement device comprising, in this order, a pump 28-1, a photoreaction unit (photoreactor 22), a pump 28-2, a gas-liquid separator 26 (with air introduction pump 28-3), and an infrared absorbance photometer 24A.

A pH buffer solution, such as a phosphoric acid buffer solution, is added using a peristaltic pump (pump 28-2) to adjust the pH to 2. Subsequently, high-purity air is mixed using pump 28-2 and introduced into the glass gas-liquid separator 26. The separated gas is introduced into the infrared absorbance photometer 24A, and measurement is performed at a wavelength of 4.3 µm using a non-dispersive infrared absorption meter. This enables the measurement of carbonate ions generated by oxidizing cyanide ions in the photoreaction unit (photoreactor 22).

The specific measurement method using the measurement device of Figure 2 will be described in Example 3 below.

### [Third Embodiment]

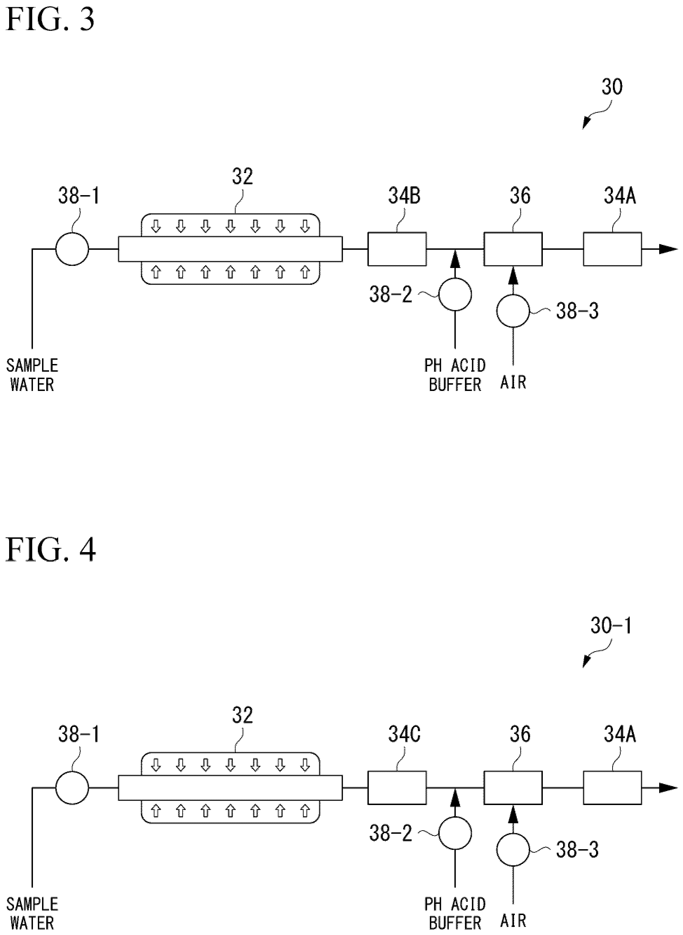

Figure 3 is a schematic diagram showing the measurement device of the third embodiment (cyanide measurement device 30).

To improve the detection selectivity for cyano, the measurement unit of the device 20 of the second embodiment shown in Figure 2 is modified by adding an ultraviolet absorbance photometer 34B, placed before the infrared absorbance photometer 34A and connected in series.

The device 30 is a flow-type measurement device comprising, in this order, a pump 38-1, a photoreaction unit (photoreactor 32), an ultraviolet absorbance photometer 34B, a pump 38-2, a gas-liquid separator 36 (with air introduction pump 38-3), and an infrared absorbance photometer 34A.

In the measurement method using the device 30 of Figure 3, since the ultraviolet and infrared detection signals are proportional to the nitrogen and carbon content of the target substance, the detection signal ratio can be used to enhance the identification accuracy of cyano.

The specific measurement method using the measurement device of Figure 3 will be described in Example 4 below.

As a variation of the third embodiment, for example, the cyanide measurement device 30-1 shown in Figure 4, which uses a conductivity detector 34C instead of the ultraviolet absorbance photometer 34B of Figure 3, may be used.

### [Fourth Embodiment]

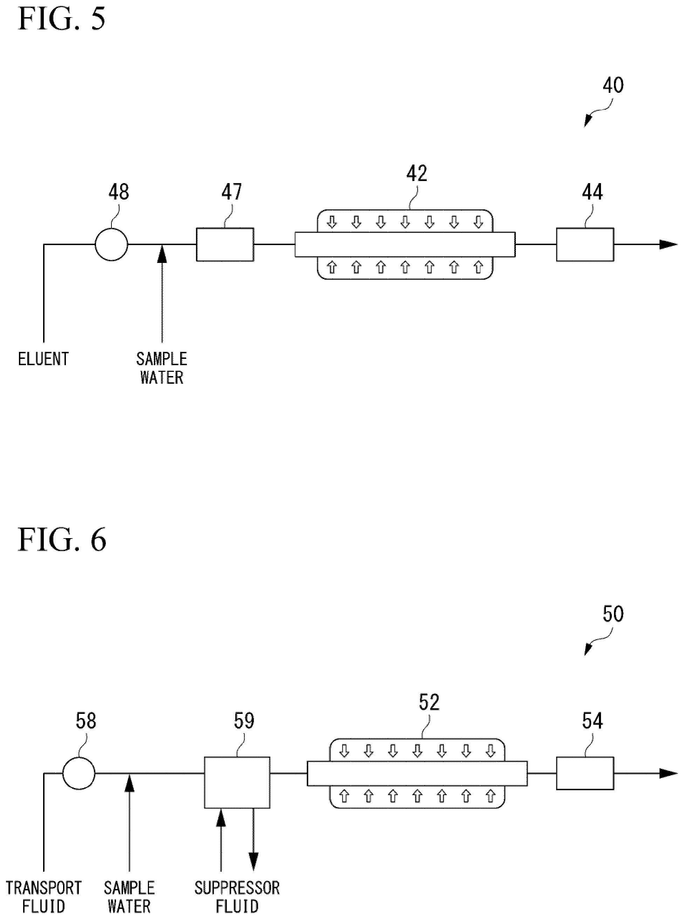

Figure 5 is a schematic diagram showing the measurement device of the fourth embodiment (cyanide measurement device 40).

The device 10 of the first embodiment shown in Figure 1 is modified by introducing a separation column 47, such as an ion chromatograph, before the photoreaction unit (photoreactor 42).

The device 40 is a flow-type measurement device comprising, in this order, a pump 48, a separation column 47, a photoreaction unit (photoreactor 42), and a measurement unit (detector 44).

When nitrate ions, nitrite ions, or carbonate ions coexist with cyano in the sample water, measuring cyano using the reagent-free photoreaction method combined with absorbance photometry become difficult. By introducing a separation column 47, such as an ion chromatograph, before the photoreaction process, cyanide (cyanide ions), nitrate ions, nitrite ions, and carbonate ions can be pre-separated, enabling the detection of cyanide by absorbance photometry.

Additionally, the separation function of the ion chromatography method (using the separation column 47) allows not only the removal of interfering nitrate ions, nitrite ions, and carbonate ions but also the simultaneous separation and measurement of other coexisting ions in the sample water, such as chloride ions or sulfate ions, along with cyano. The detector 44 can be any method that detects nitrate ions, nitrite ions, or carbonate ions after the photoreaction process of cyano, including ultraviolet absorbance photometry, infrared absorbance photometry, or conductivity detection. In the case of infrared absorbance photometry, if carbonate ions originally present in the sample water interfere with measurement, the conditions of the ion chromatograph (separation column 47) can be adjusted for separation. Furthermore, combining conductivity detection with a suppressor method enables higher sensitivity by converting nitrate ions, nitrite ions, and carbonate ions into hydroxide ions.

The specific measurement method using the measurement device of Figure 5 will be described in Example 5 below.

### [Fifth Embodiment]

Figure 6 is a schematic diagram showing the measurement device of the fifth embodiment (cyanide measurement device 50).

The device 40 of the fourth embodiment shown in Figure 5 is modified by using a suppressor 59 instead of the separation column 47, such as an ion chromatograph. Nitrate ions, nitrite ions, and carbonate ions coexisting in the sample water are pre-converted into hydroxide ions, while cyano is retained as undissociated hydrogen cyanide during the photoreaction process. The resulting nitrate ions, nitrite ions, and carbonate ions can then be detected by ultraviolet absorbance photometry, infrared absorbance photometry, or conductivity detection.

The device 50 in Figure 6 is a flow-type measurement device comprising, in this order, a pump 58, a suppressor 59, a photoreaction unit (photoreactor 52), and a measurement unit (detector 54).

For example, as one instance, the sample water is introduced into an electrolytic suppressor (suppressor 59) using a weakly alkaline aqueous solution as the carrier liquid. Water is also introduced as the suppressor solution. A voltage is applied to the electrodes within the suppressor 59, generating hydroxide ions that are exchanged with nitrate ions, nitrite ions, and carbonate ions in the sample water via an anion exchange membrane. Meanwhile, cyanois retained as hydrogen cyanide and introduced into the photoreactor 52. Additionally, combining the suppressor 59 with conductivity detection enables enhanced detection sensitivity.

An example of the suppressor includes the DRS-600 manufactured by Thermo Scientific.

### [Sixth Embodiment]

The procedure for quantifying a nitrogen/carbon-containing compound in an aqueous solution and the procedure for measuring the nitrogen content and carbon content of an unknown compound (e.g., p-nitrophenol) will be described.

The measurement method for a nitrogen/carbon-containing compound according to the present embodiment can combine a method for detecting nitrate ions and nitrite ions with a method for detecting carbonate ions or carbon dioxide, generated by the vacuum ultraviolet light reaction process. In this method, by using an aqueous solution of a nitrogen/carbon-containing compound with a known chemical composition as a standard solution, it is possible to quantify nitrogen/carbon-containing compounds with different chemical compositions in the aqueous solution.

An example of the device is the device 30 of the third embodiment shown in Figure 3. For instance, sodium cyanide is used as the compound of the standard solution, and p-nitrophenol is the compound to be quantified. Measurements are performed using aqueous solutions with varying cyano concentrations as the standard solution, and calibration curves are created based on the relationship between the detection signal values obtained by ultraviolet and infrared absorbance photometry and the nitrogen or carbon concentrations.

Next, an aqueous solution containing the compound to be quantified is measured as the sample water, and the nitrogen and carbon concentrations of the compound in the sample water are calculated from the detection signal values obtained by each absorbance photometry method and the calibration curves. By dividing these nitrogen or carbon concentrations by the nitrogen content or carbon content of the compound (nitrogen: 51.8%, carbon: 10.0%), the concentration of the nitrogen/carbon-containing compound in the sample water is determined.

Furthermore, this quantitative method using a standard solution of a single nitrogen/carbon-containing compound can also determine the nitrogen and carbon content of an unknown nitrogen/carbon-containing compound.

An example of the device is the device 30 of Figure 3. As a measurement example, sodium cyanide is used as the compound of the standard solution. Calibration curves for ultraviolet and infrared absorbance photometry are created following the same procedure as the quantitative method described above. Next, an aqueous solution containing a known amount of the unknown nitrogen/carbon-containing compound is measured as the sample water, and the nitrogen and carbon concentrations of the unknown compound in the sample water are calculated from the detection signal values obtained by each absorbance photometry method and the calibration curves. By dividing these nitrogen or carbon concentrations by the concentration of the compound in the sample water, the nitrogen content and carbon content of the unknown compound are determined.

### [Seventh Embodiment]

In Figure 1, Figure 2, Figures 3 and 4, Figure 5, and Figure 6, which illustrate the first to fifth embodiments, respectively, each photoreactor 12, 22, 32, 42, and 52 may further include a temperature control unit. Preferably, the temperature control unit includes a temperature measurement device for measuring the temperature of the photoreactor or the sample water and a cooling device for cooling the photoreactor. The temperature measurement device may include, for example, one or more temperature sensors, such as thermocouple temperature sensors. The cooling device may include, for example, cooling means such as an exhaust fan or Peltier element, either alone or in combination.

By installing the temperature control unit in the photoreactor, the photoreaction efficiency can be enhanced, improving the measurement sensitivity and speed. For example, when a 6-watt mercury lamp and a built-in reaction tube (inner diameter: 2 mm, length: 135 mm) are used as the photoreactor with the temperature control unit, acetic acid sample water (carbon concentration: 2 ppm) at 25°C is flowed through the reaction tube at a flow rate of 1.6 mL/min. In this case, the heat generated by the mercury lamp can raise the sample water temperature to 40°C at the reaction tube outlet. By using the cooling device of the temperature control unit, the sample water can be maintained at 40°C. As a result, a conversion rate of 88% to carbonate ions is achieved with a reaction time of 1.1 minutes.

Another example of installing the temperature control unit in the photoreactor will be described. For instance, the built-in reaction tube photoreactor comprises a 40-watt mercury lamp and a quartz tube with an inner diameter of 2 mm and a length of 290 mm. The cooling device may have a structure in which the mercury lamp is closely attached to an aluminum block, with two Peltier elements equipped with exhaust fans installed in parallel along the longitudinal direction of the reaction tube on one side of the block. The temperature measurement device may include two thermocouple temperature sensors, which are inserted through the top of the aluminum block at a position 2 cm from both ends of the mercury lamp, contacting the outer side of the lamp. The temperature control unit comprises the temperature measurement device, the cooling device, and a temperature controller. By connecting the temperature measurement device and the cooling device to the temperature controller, the sensor temperature of the temperature measurement device is measured, and the Peltier elements of the cooling device are cooled or heated to maintain the set temperature, thereby controlling the temperature. An example of temperature control is described below.

For example, when the temperature sensors A and B outside the lamp are set to 30°C and cooled, a constant temperature of 30°C ± 1°C can be maintained 30 minutes after the lamp is turned on. As a result, the flow rate of the sample solution is stabilized. For instance, when an aqueous solution containing 1 ppm of cyano is delivered at a flow rate of 0.4 mL/min, a coefficient of variation of 1% is achieved, ensuring stable delivery. The total conversion rate from cyano to nitrate ions and nitrite ions is 96%, achieving near-complete conversion and high photoreaction efficiency.

As an example of measurement stability, sample water containing 1 ppm of cyanide ions is introduced into the photoreactor (40 watts, 2 mm) at a flow rate of 1.2 mL/min using the pump described above. When absorbance at a wavelength of 220 nm is continuously measured for 20 minutes using a flow-type ultraviolet absorbance photometer SPDM10A manufactured by Shimadzu, the coefficient of variation is 1%. Stable measurement of cyano is also achieved.

The cooling means of the cooling device is not limited to a specific method as long as cooling is possible and may include Peltier cooling, water cooling, or air cooling. The position of the temperature sensor in the temperature measurement device may be any position that allows control, such as outside the lamp, inside the reaction tube, or at the reaction tube outlet.

### Examples

Hereinafter, the present invention will be described in further detail with reference to examples. However, the present invention is not limited to these examples in any way.

### (Raw Materials Used)

Cyanide ion standard solution: Manufactured by Kanto Chemical Co., Inc., concentration: 1,000 ppm
Nitrite ion standard solution: Manufactured by Kanto Chemical Co., Inc., concentration: 1,000 ppm
Nitrate ion standard solution: Manufactured by Kanto Chemical Co., Inc., concentration: 1,000 ppm
Potassium hexacyanocobaltate(III): Manufactured by Kanto Chemical Co., Inc.
Nitrophenol: Manufactured by Kanto Chemical Co., Inc.

### (Equipment Used)

Built-in reaction tube photoreactor incorporated within the lamp:
Low-pressure mercury lamp: 40 watts or 20 watts, illuminance at 185 nm: 9 mW/cm² for 40 watts
Quartz reaction tube: Made of synthetic fused silica, size: inner diameter 2 mm × length 290 mm, installed coaxially within the mercury lamp tube.

Flow-type ultraviolet absorbance photometer: SPD-10Avp, manufactured by Shimadzu Corporation
Flow-type conductivity detector: CDD-10Avp, manufactured by Shimadzu Corporation
Non-dispersive infrared absorption meter: Model 840, manufactured by Nicolet Corporation
Ion chromatograph: Model 1260, manufactured by Agilent Technologies
   Anion exchange column: AS-15, manufactured by Thermo Scientific
Glass gas-liquid separator: Double-tube type, made of glass
Peristaltic tube pump: MINIPULS 3, manufactured by Gilson, Inc.
Teflon (registered trademark) tube: Inner diameter 0.5 mm × 1 m, manufactured by GL Sciences Inc.

### (Example 1)

Measurements were performed using the cyanide measurement device 10 shown in Figure 1.

The device 10 includes a peristaltic tube pump, a built-in reaction tube photoreactor using a 40-watt low-pressure mercury lamp as the light source with a quartz reaction tube incorporated within the lamp, and a flow-type ultraviolet absorbance photometer as the detector 14, and the components were connected by Teflon tubes.

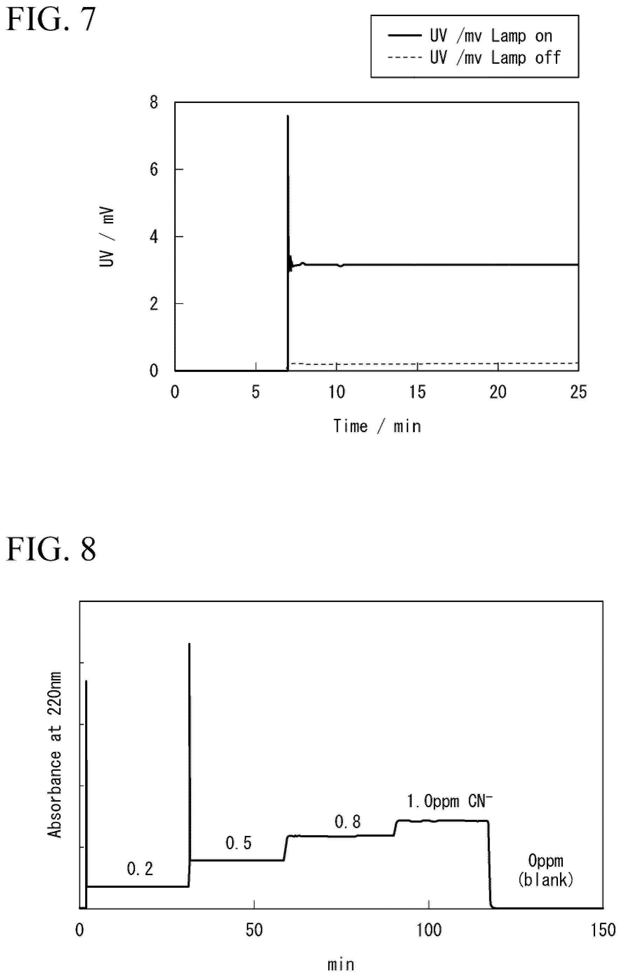

Using this device, an aqueous solution of sodium cyanide containing 1.0 ppm of cyano was used as the test sample water and introduced online into the built-in reaction tube vacuum ultraviolet photoreactor via the pump. After 0.7 minutes of irradiation, the solution was introduced online into the ultraviolet absorbance photometer, and absorbance was measured at a wavelength of 220 nm. The measurement results are shown in Figure 7. Initially, pure water was introduced, and the test sample water was introduced 5 minutes after the start of measurement.

Test sample water with cyano concentrations of 0.2 ppm, 0.5 ppm, 0.8 ppm, 1.0 ppm, and 0 ppm was introduced online for 30 or 40 minutes each, and measurements were performed in the same manner as described above.

The absorbance profiles of the calibration curves for cyano concentrations of 0.2 ppm, 0.5 ppm, 0.8 ppm, 1.0 ppm, and 0 ppm are shown in Figure 8.

### (Comparative Example 1)

Measurements were performed in the same manner as in Example 1, except that irradiation was performed for 0 minutes (lamp OFF) instead of 0.7 minutes. The results are shown in Figure 7.

### (Discussion of Example 1 and Comparative Example 1)

As shown in Figures 7 and 8, the photoreaction process increased the detection sensitivity by 16 times, enabling high-sensitivity detection. Additionally, the calibration curve in the range of 0 to 1.0 ppm exhibited excellent linearity with a correlation coefficient of 0.999 or higher, achieving quantitative measurement.

The components in the photoreaction-treated liquid were analyzed by ion chromatography. As a result, it was confirmed that 89% of cyano was converted to nitrate ions and nitrite ions through a short, reagent-free photoreaction process of 0.7 minutes. These nitrogen oxoanions, which exhibit ultraviolet absorption, enabled high-sensitivity detection. Furthermore, the conversion rate can be improved by extending the reaction time. When the flow rate was reduced to extend the reaction time to 1.0 minute, 97% of 1 ppm cyano was converted to nitrate ions and nitrite ions.

### (Reference Example 1)

In addition to the cyanide ions in Example 1, highly refractory heavy metal cyano complex compounds can also be rapidly converted.

The cyanide measurement device 10 shown in Figure 1, used a peristaltic tube pump, a built-in reaction tube photoreactor using a 40-watt low-pressure mercury lamp as the light source with a quartz reaction tube incorporated within the lamp, and the components were connected by Teflon tubes.

By using the above-mentioned device, an aqueous solution of potassium hexacyanocobaltate(III) containing 1.0 ppm of cyano was used as the test sample water. The solution was introduced online into the built-in reaction tube vacuum ultraviolet photoreactor via the pump, and the solution after 4.8 minutes of irradiation was analyzed by ion chromatography.

As a result, 97% of cyano was converted to nitrate ions.

Cobalt cyano complexes have an extremely high stability constant of 10⁶⁴, making them highly refractory among cyanides. However, since rapid and near-complete conversion to nitrate ions is possible, it was confirmed that various cyanides can be detected and quantified using inexpensive absorbance photometry without the use of colorimetric reagents or electrodes, similar to the detection of cyanide salts described above.

### (Reference Example 2)

It is further demonstrated that conversion is possible for nitrogen/carbon-containing compounds other than cyanide in Example 1.

The cyanide measurement device 10 shown in Figure 1, used a peristaltic tube pump, a built-in reaction tube photoreactor using a 20-watt low-pressure mercury lamp as the light source with a quartz reaction tube incorporated within the lamp, and the components were connected by Teflon tubes.

By using the above-mentioned device, an aqueous solution of p-nitrophenol containing 1.0 ppm was used as the test sample water. The solution was introduced online into the built-in reaction tube vacuum ultraviolet photoreactor via the pump, and the solution after 0.6 minutes of irradiation was analyzed by ion chromatography.

As a result, 88% of the nitrogen content of p-nitrophenol was converted to nitrate ions.

Since nitrogen/carbon-containing compounds other than cyanide can also be converted in this manner, it was confirmed that they can be detected and quantified using inexpensive absorbance photometry without the use of colorimetric reagents or electrodes, similar to the cyanide described above.

### (Example 2)

The measurement method of Example 1 can be applied to methods other than absorbance photometry, as long as they can detect nitrate ions or nitrite ions. Here, a measurement method using a conductivity detector is described.

Measurements were performed using the cyanide measurement device 10 shown in Figure 1.

The device 10 had the same configuration as in Example 1, except that a flow-type conductivity detector was used as the detector 14. The device 10 used a peristaltic tube pump, a built-in reaction tube photoreactor using a 40-watt low-pressure mercury lamp as the light source with a quartz reaction tube incorporated within the lamp, and a flow-type conductivity detector, and the components were connected by Teflon tubes.

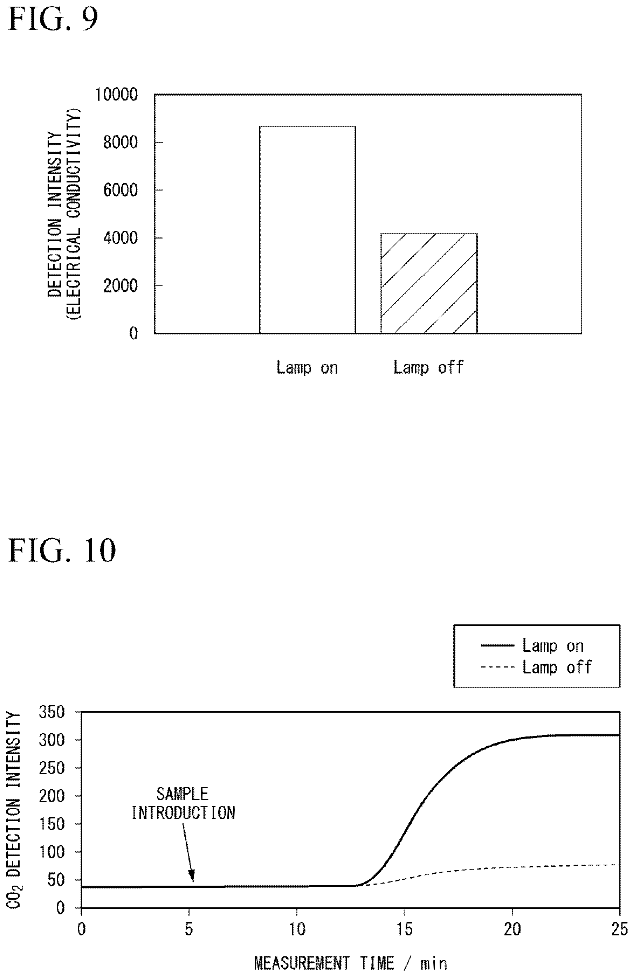

By using the above-mentioned device, an aqueous solution of potassium hexacyanocobaltate(III) containing 1.0 ppm of cyano was used as the test sample water. The test sample was introduced online into the built-in reaction tube vacuum ultraviolet photoreactor via the pump, and the solution after 2.6 minutes of irradiation was introduced online into the conductivity detector for conductivity measurement. The measurement results are shown in Figure 9.

### (Comparative Example 2)

Measurements were performed in the same manner as in Example 2, except that irradiation was performed for 0 minutes (lamp OFF) instead of 2.6 minutes. The results are shown in Figure 9.

### (Discussion of Example 2 and Comparative Example 2)

As shown in Figure 9, the photoreaction process increased the detection sensitivity by 2.0 times, enabling high-sensitivity detection. The sensitization effect is more significant for cyanides with lower electrical conductivity, such as those in Example 2, compared to compounds with high electrical conductivity, such as cobalt cyano complex compounds. Furthermore, adding a suppressor before the conductivity detector can further enhance detection sensitivity.

### (Example 3)

Measurements were performed using the measurement device 20 shown in Figure 2.

The components of device 20 up to the photoreactor 22 are the same as those in Figure 1. However, after the photoreactor 22, a pH buffer solution, specifically a phosphoric acid buffer solution, was added using a peristaltic pump (pump 28-2) to adjust the pH to 2. Subsequently, high-purity air was mixed using pump 28-3 and introduced into a glass gas-liquid separator 26. The separated gas was introduced into an infrared absorbance photometer 24A, and measurements were performed at a wavelength of 4.3 µm using a non-dispersive infrared absorption meter. The test sample and irradiation conditions were the same as those in Example 1. Initially, pure water was delivered by the pump, followed by the test sample. The measurement results are shown in Figure 10.

### (Comparative Example 3)

Measurements were performed in the same manner as in Example 3, except that irradiation was performed for 0 minutes (lamp OFF) instead of 0.7 minutes. The results are shown in Figure 10.

### (Discussion of Example 3 and Comparative Example 3)

The reagent-free photoreaction method generated carbonate ions from cyan, which were converted to carbon dioxide by adjusting the solution to pH 2, enabling high-sensitivity detection with an infrared absorbance photometer.

### (Reference Example 3)

It is further demonstrated that nitrogen/carbon-containing compounds other than cyanide in Example 3 can also be converted to carbonate ions.

The cyanide measurement device 10 shown in Figure 1 used a peristaltic tube pump, and a built-in reaction tube photoreactor using a 6-watt low-pressure mercury lamp as the light source with a quartz reaction tube incorporated within the lamp, and the components were connected by Teflon tubes.

By using the above-mentioned device, aqueous solutions of disodium ethylenediaminetetraacetic acid (EDTA) (C₁₀H₁₄N₂Na₂O₈) with a carbon concentration of 2.0 ppm (mg of carbon/L) and disodium lead ethylenediaminetetraacetic acid (EDTA) (C₁₀H₁₂N₂Na₂O₈Pb) with a carbon concentration of 2.0 ppm (mg of carbon/L, aqueous solution concentration of lead EDTA: 17 µmol/L) were used as test sample water. Each test sample was introduced online into the built-in reaction tube vacuum ultraviolet photoreactor via the pump, and the solution after 1.1 minutes of irradiation was analyzed using a total organic carbon analyzer TOC-V (manufactured by Shimadzu Corporation) to measure the residual organic carbon concentration.

As a result, no residual organic carbon was detected for either of the two EDTA compounds, with concentrations below the limit of detection of 0.08 ppm (mg of carbon/L) of the total organic carbon analyzer. This indicates that 96% or more of the carbon content of each EDTA compound was mineralized and converted to carbonate ions.

Thus, since nitrogen/carbon-containing compounds other than cyanide can also be converted in this manner, it is evident that they can be detected and quantified using inexpensive infrared absorbance photometry without the use of colorimetric reagents or electrodes, similar to the cyanide described above.

### (Example 4)

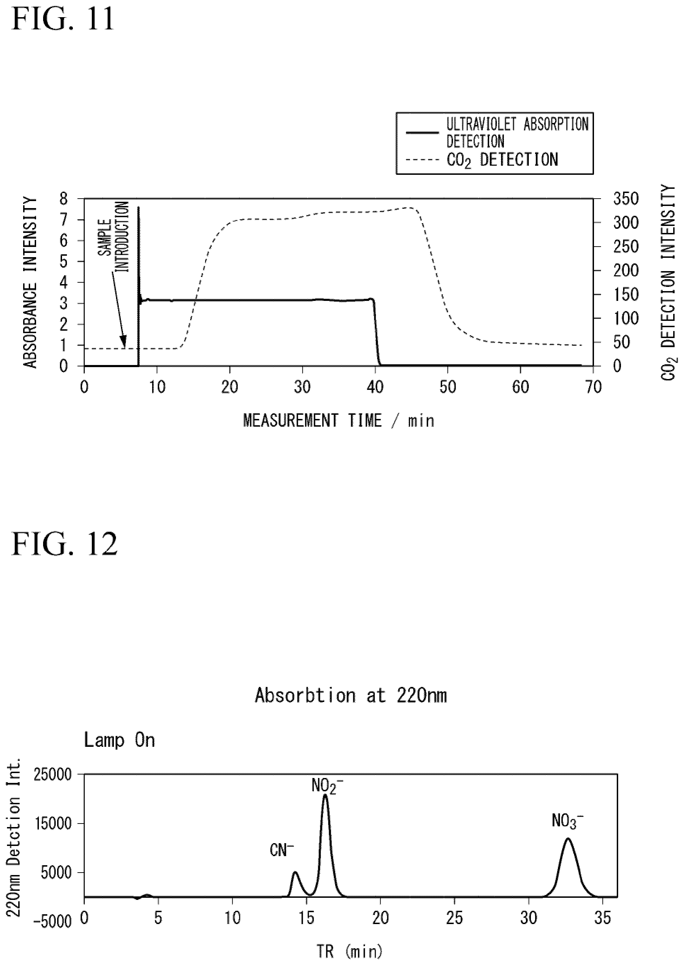

Measurements were performed using the measurement device 30 shown in Figure 3. Using the same irradiation conditions and sample preparation conditions as in Example 1, the measurement results for 1.0 ppm cyano are shown in Figure 11. Both ultraviolet and infrared absorbance achieved high-sensitivity detection. Since the ultraviolet and infrared detection signals are proportional to the nitrogen and carbon content of the target substance, the detection signal ratio can be used to enhance the identification accuracy of cyano.

### (Example 5)

Measurements were performed using the device 40 shown in Figure 5.

The detector 44 was a flow-type ultraviolet absorbance photometer. The ion chromatograph (separation column 47) used an aqueous potassium hydroxide solution as the eluent and an anion exchange column for component separation. The column outlet and the inlet of the photoreactor 42 were connected with a Teflon tube. The test sample water was a mixed solution containing 2.7 ppm cyan, 5 ppm nitrite ions, and 5 ppm nitrate ions (concentrations based on nitrogen content), introduced into the ion chromatograph via an injector for measurement. The irradiation conditions were the same as those in Example 1. The measurement results are shown in Figure 12.

### (Comparative Example 4)

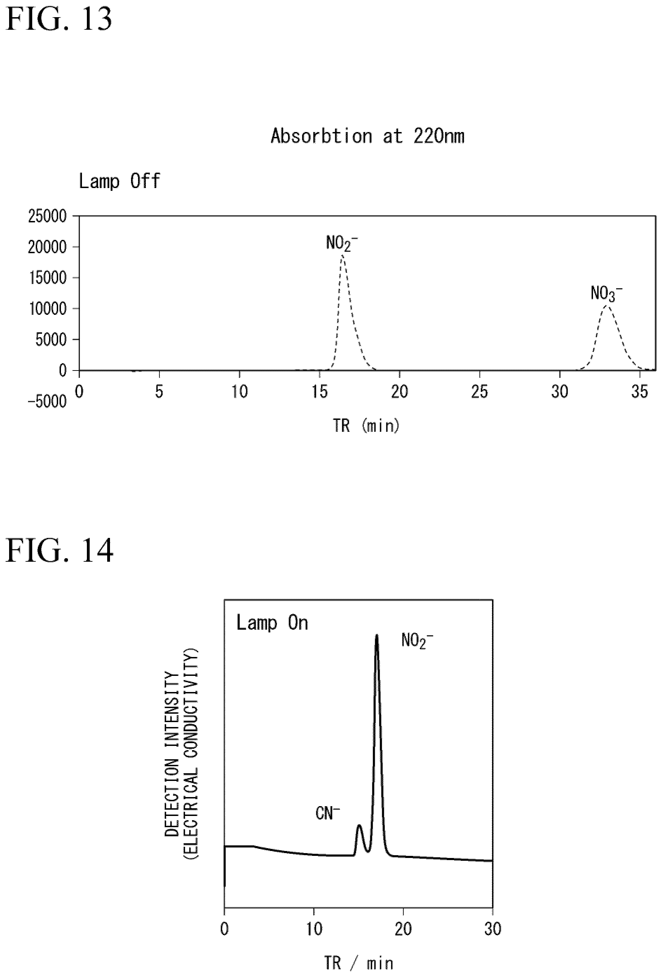

Measurements were performed in the same manner as in Example 5, except that irradiation was performed for 0 minutes (lamp OFF) instead of 0.7 minutes. The results are shown in Figure 13.

### (Discussion of Example 5 and Comparative Example 4)

Even when nitrate ions and nitrite ions, which interfere with measurement by ultraviolet absorbance photometry, are present in the sample water, cyano can be separated and detected.

### (Example 6)

Measurements were performed using the device 40 shown in Figure 5.

The detector 44 was a conductivity detector with a suppressor. The ion chromatograph (separation column 47) used an aqueous potassium hydroxide solution as the eluent and an anion exchange column for component separation. The column outlet and the inlet of the photoreactor 42 were connected with a Teflon tube. The test sample water which was a mixed solution containing 2.7 ppm cyano and 5 ppm nitrite ions (concentrations based on nitrogen content), was introduced into the ion chromatograph via an injector for measurement. The irradiation conditions were the same as those in Example 1. The measurement results are shown in Figure 14.

### (Comparative Example 5)

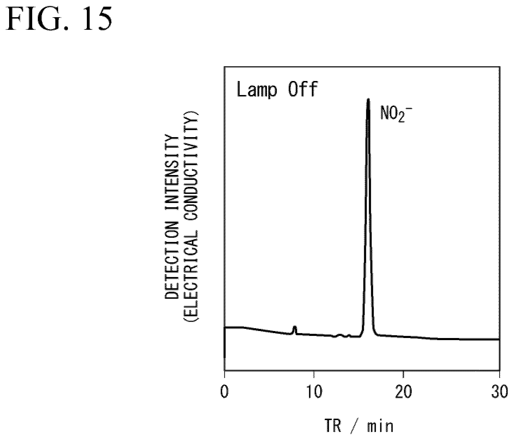

Measurements were performed in the same manner as in Example 6, except that the photoreactor was omitted from the device in Figure 5. The results are shown in Figure 15.

### (Discussion of Example 6 and Comparative Example 5)

Even when nitrite ions, which interfere with measurement by conductivity detection, are present in the sample water, cyano can be separated and detected.

### INDUSTRIAL APPLICABILITY

One embodiment of the present invention provides a measurement method and device enabling high-sensitivity and high-selectivity simultaneous measurement of carbon and nitrogen using an inexpensive detection method. In particular, for cyanides, which have few properties allowing high-sensitivity and selective detection, a measurement method and device enabling high-sensitivity and high-selectivity measurement can be provided. The measurement method and device of the present invention can be used in the following industrial fields: devices can be used in any fields requiring the analysis and management of cyan, such as environmental water conservation, management of tap water, industrial water, and wastewater, water treatment, biological treatment; existing cyano analysis devices (ion chromatographs, flow analysis systems), and on-site monitoring devices.

### REFERENCE SIGNS LIST

10, 20, 30, 30-1, 40, 50: Cyanide measurement device
12, 22, 32, 42, 52: Photoreactor
14, 44, 54: Detector
24A, 34A: Infrared absorbance photometer
34B: Ultraviolet absorbance photometer
34C: Conductivity detector
26, 36: Gas-liquid separator
47: Separation column
18, 28-1, 28-2, 28-3, 38-1, 38-2, 38-3, 48, 58: Pump
59: Suppressor

## Claims

1. A method for measuring a nitrogen/carbon-containing compound comprising at least one element selected from the group consisting of nitrogen and carbon, the method comprising:
a photoreaction step of irradiating an aqueous solution containing the nitrogen/carbon-containing compound with light having a wavelength of 190 nm or less to generate analyte ions; and
a measurement step of measuring the analyte ions,
wherein the analyte ions are at least one ion selected from the group consisting of nitrate ions, nitrite ions, and carbonate ions.

2. The method for measuring a nitrogen/carbon-containing compound according to claim 1, wherein the nitrogen/carbon-containing compound is cyanide.

3. The method for measuring a nitrogen/carbon-containing compound according to claim 1 or 2, wherein the measurement step comprises a nitrogen component measurement step of measuring the nitrate ions and the nitrite ions by ultraviolet spectrophotometry.

4. The method for measuring a nitrogen/carbon-containing compound according to claim 1 or 2, wherein the measurement step comprises a nitrogen component measurement step of measuring the nitrate ions and the nitrite ions by electrical conductivity measurement.

5. The method for measuring a nitrogen/carbon-containing compound according to claim 1 or 2, wherein the measurement step comprises a carbon component measurement step of converting the carbonate ions into carbon dioxide and measuring the carbon dioxide by infrared spectrophotometry.

6. A measurement device for measuring a nitrogen/carbon-containing compound comprising at least one element selected from the group consisting of nitrogen and carbon, the device comprising:
a photoreaction unit and a measurement unit,
wherein the photoreaction unit comprises a reaction vessel and a light source, the light source irradiating light having a wavelength of 190 nm or less,
the photoreaction unit generates analyte ions from an aqueous solution containing the nitrogen/carbon-containing compound,
the measurement unit measures the analyte ions, and
the analyte ions are at least one ion selected from the group consisting of nitrate ions, nitrite ions, and carbonate ions.

7. The measurement device for a nitrogen/carbon-containing compound according to claim 6, wherein the nitrogen/carbon-containing compound is cyanide.

8. The measurement device for a nitrogen/carbon-containing compound according to claim 6 or 7, wherein the measurement unit comprises a nitrogen component measurement unit comprising an ultraviolet spectrophotometer.

9. The measurement device for a nitrogen/carbon-containing compound according to claim 6 or 7, wherein the measurement unit comprises a nitrogen component measurement unit comprising an electrical conductivity detector.

10. The measurement device for a nitrogen/carbon-containing compound according to claim 6 or 7, wherein the measurement unit comprises a carbon component measurement unit comprising a conversion unit for converting the carbonate ions into carbon dioxide and an infrared spectrophotometer.

11. The measurement device for a nitrogen/carbon-containing compound according to claim 6 or 7, wherein the measurement device is a flow-type measurement device further comprising a liquid delivery unit and a sample introduction unit, and the liquid delivery unit, the sample introduction unit, the photoreaction unit, and the measurement unit are arranged in this order.

12. The measurement device for a nitrogen/carbon-containing compound according to claim 6 or 7, wherein the measurement device is a flow-type measurement device further comprising a liquid delivery unit, a sample introduction unit, and a separation column, and the liquid delivery unit, the sample introduction unit, the separation column, the photoreaction unit, and the measurement unit are arranged in this order.

13. The measurement device for a nitrogen/carbon-containing compound according to claim 6 or 7, wherein the measurement device is a flow-type measurement device further comprising a liquid delivery unit, a sample introduction unit, and a suppressor, and the liquid delivery unit, the sample introduction unit, the suppressor, the photoreaction unit, and the measurement unit are arranged in this order.

14. The measurement device for a nitrogen/carbon-containing compound according to claim 6 or 7, wherein the photoreaction unit further comprises a temperature control unit.

15. The measurement device for a nitrogen/carbon-containing compound according to claim 14, wherein the temperature control unit comprises a temperature measurement device for measuring the temperature of the photoreaction unit and a cooling device for cooling the photoreaction unit, and wherein the temperature of the photoreaction unit is at least one temperature selected from the group consisting of the temperature of the reaction vessel, the temperature of the light source, and the temperature of the aqueous solution.
